# EUROPEAN PATENT APPLICATION

(11) **EP 3 431 594 A1**
(43) Date of publication of application: **23.01.2019**
(21) Application number: 17766592.4
(22) Date of filing: 13.03.2017
(51) Int. Cl.: C12N 15/09, C12Q 1/68, G01N 33/53

(54) **OBESITY RISK DIAGNOSIS KIT AND METHOD FOR ANALYZING RISK OF OBESITY ONSET**

(30) Priority: 15.03.2016 JP 2016051038
(71) Applicant: Watahiki, Hajime, Minato-ku, Tokyo 108-0075 (JP); Nakajima, Toshihiro, Aoba-ku Yokohama-shi Kanagawa 225-0023 (JP)
(72) Inventor: NAKAJIMA, Toshihiro, Yokohama-shi Kanagawa 225-0023 (JP); ARATANI, Satoko, Tokyo 160-8402 (JP); FUJITA, Hidetoshi, Tokyo 160-8402 (JP)
(74) Representative: Simpson, Tobias Rutger
(86) International application number: PCT/JP2017/009903
(87) International publication number: WO 2017/159592

(57) **Abstract**

[Problem] To provide an obesity risk diagnosis kit and a method for analyzing the risk of obesity onset. [Solution] An obesity risk diagnosis kit including a probe for analyzing genetic information of an obese person and a non-obese person and identifying a synoviolin gene as an obesity marker by utilizing elevated expression of the synoviolin gene in the obese person. A method for analyzing the risk of obesity onset, including a step for measuring the expression level of the synoviolin gene in a sample collected from a subject organism, and a step for determining the risk of obesity onset using the expression level of the synoviolin gene.

SEQUENCE LISTING

<110>  Watahiki, Hajime
       Nakajima, Toshihiro

<120>  Kit for diagnosis of the risk of obesity and method of analysing
       the risk of obesity

<130>  15-283P

<160>  15

<170>  PatentIn version 3.5

<210>  1
<211>  123
<212>  DNA
<213>  Homo sapiens

<400>  1
ccagtacctc accgtgctgg cctccttggg gcccccccgg cctgccactt cagtcaactc      60

cactgaggag actgccacta cagttgttgc tgctgcctcc tccaccagca tccctagctc     120

aga                                                                   123


<210>  2
<211>  19
<212>  DNA
<213>  Artificial Sequence

<220>
<223>  primer

<400>  2
ccagtacctc accgtgctg                                                   19


<210>  3
<211>  20
<212>  DNA
<213>  Artificial Sequence

<220>
<223>  primer

<400>  3
tctgagctag ggatgctggt                                                  20


<210>  4
<211>  20
<212>  DNA
<213>  Artificial Sequence

<220>
<223>  primer

<400>  4
gcagaagggg agacacattt                                                  20

<210>  5
<211>  21
<212>  DNA
<213>  Artificial Sequence

<220>
<223>  primer

<400>  5
tgtggtcttg ttatgggtgg t                                                21


<210>  6
<211>  20
<212>  DNA
<213>  Artificial Sequence

<220>
<223>  primer

<400>  6
ggagttaaga cagcgcttgg                                                  20


<210>  7
<211>  20
<212>  DNA
<213>  Artificial Sequence

<220>
<223>  primer

<400>  7
cactggcctc acttcattcc                                                  20


<210>  8
<211>  20
<212>  DNA
<213>  Artificial Sequence

<220>
<223>  primer

<400>  8
gaagcatgtg ctcaaacacc                                                  20


<210>  9
<211>  18
<212>  DNA
<213>  Artificial Sequence

<220>
<223>  primer

<400>  9
tctgtcgctc acgtcctg                                                    18


<210>  10
<211>  22
<212>  DNA

<213>  Artificial Sequence

<220>
<223>  primer

<400>  10
gaagctaaga aagctgcaaa gc                                                22


<210>  11
<211>  20
<212>  DNA
<213>  Artificial Sequence

<220>
<223>  primer

<400>  11
cagtgtttcg tggtgtgctc                                                   20


<210>  12
<211>  20
<212>  DNA
<213>  Artificial Sequence

<220>
<223>  primer

<400>  12
catcaagttc ttgccgttca                                                   20


<210>  13
<211>  23
<212>  DNA
<213>  Artificial Sequence

<220>
<223>  primer

<400>  13
ttcaggagca aatgtctttg ttt                                               23


<210>  14
<211>  20
<212>  DNA
<213>  Artificial Sequence

<220>
<223>  primer

<400>  14
gcagaagggg agacacattt                                                   20


<210>  15
<211>  21
<212>  DNA
<213>  Artificial Sequence

<220>

<223>  primer

<400>  15
tgtggtcttg ttatgggtgg t                                                21

## Description

### Technical Field

The present invention relates to an obesity risk diagnosis kit and a method for analyzing the risk of obesity onset.

### Background Art

JP 5279492 B2 discloses a probe which is used for analyzing a polymorphism of an obesity gene. As described above, the technology for analyzing the obesity gene so as to analyze the genetic risk of a subject developing obesity has been developed.

On the other hand, WO 2014-103863 A describes that obesity can be prevented by suppressing the expression of synoviolin.

### Citation List

### Patent Literature

Patent Literature 1: JP 5279492 B2
Patent Literature 1: WO 2014-103863 A

### Summary of Invention

### Technical Problem

The present invention provides an obesity risk diagnosis kit and a method for analyzing the risk of obesity onset.

### Solution to Problem

Basically, the present invention is based on the findings that genetic information on an obese person and a non-obese person is analyzed, and that the obese person expresses a large number of *SYNOVIOLIN* genes in an example. That is, the expression level of the *SYNOVIOLIN* gene of the obese person is higher than the expression level of the *SYNOVIOLIN* gene of the non-obese person, with a statistically significant difference. Therefore, it is considered that the *SYNOVIOLIN* gene can be an obesity marker for judging the risk (possibility of morbidity) as to whether or not a person tends to be genetically obese. Further, the expression level of the *SYNOVIOLIN* gene of the subject is determined and compared with a predetermined value (a threshold value related to the expression level of the *SYNOVIOLIN* gene which has been measured and determined in advance) so that it is possible to analyze whether the subject is at risk of genetically developing obesity.

A first aspect of the present invention relates to an obesity risk diagnosis kit. The obesity risk diagnosis kit includes a probe for identifying a *SYNOVIOLIN* gene as an obesity marker.

A second aspect of the present invention relates to a method for analyzing the risk of obesity onset. This method includes the steps of measuring the expression level of a *SYNOVIOLIN* gene in a sample collected from an organism as a subject and determining the risk of obesity onset using the expression level of the *SYNOVIOLIN* gene.

### Advantageous Effects of Invention

The present invention can provide an obesity risk diagnosis kit and a method for analyzing the risk of obesity onset.

### Brief Description of Drawings

Fig. 1 is a box plot showing the expression levels of the *SYNOVIOLIN* gene and the *ATF 6* gene.
Fig. 2 is a box plot showing the expression levels of the *XBP1* gene and the *elF2* gene.
Fig. 3 is a box plot showing the expression levels of the *GRP78* gene and the *IRE1* gene.

### Description of Embodiments

Description of embodiments of the present invention will be given hereinafter with reference to the drawings. The present invention is not limited to the embodiments described below, but includes modifications appropriately modified by a person skilled in the art in a range obvious from the embodiments below.

A first aspect of the present invention relates to an obesity risk diagnosis kit. In this diagnosis kit, a sample (e.g., blood, saliva, nail, hair) is collected from an organism (e.g., a human) as a subject and the expression level of the *SYNOVIOLIN* gene contained in the collected sample is measured, thereby analyzing and evaluating whether the subject may be genetically obese (obesity risk). Therefore, the obesity risk diagnosis kit includes a probe for identifying a *SYNOVIOLIN* gene as an obesity marker. This probe is used so that the expression level of the *SYNOVIOLIN* gene can be determined. This diagnosis kit can be used for genetic diagnosis, such as determining whether the organism has a gene which is likely to become obesity. In addition to the probe, the diagnosis kit may include known elements for measuring the expression level of the *SYNOVIOLIN* gene of the subject. Such elements are, for example, primers used for PCR and may include PCR measuring devices.

As the method for determining the expression level of the *SYNOVIOLIN* gene, a known method may be appropriately adopted. The method for determining the expression level of the *SYNOVIOLIN* gene is, for example, PCR. The expression level of the *SYNOVIOLIN* gene may be measured, for example, at the mRNA level, or may be measured at the protein level. Examples of the method for determining the expression level of the *SYNOVIOLIN* gene include Northern blotting, quantitative RT-PCR, Western blotting, ELISA, and immunostaining. Since these methods are publicly known and kits for realizing these methods are on the market, the expression level of the *SYNOVIOLIN* gene can be determined by referring to the handling manuals of the kits.

The probe for identifying the *SYNOVIOLIN* gene is known. An example of the probe for identifying the *SYNOVIOLIN* gene is the probe No. 16 of the Universal ProbeLibrary (Roche). The region amplified with this probe is that of SEQ ID NO: 1.
SEQ ID NO: 1:

ccagtacctcaccgtgctg,gcctccttggggccccccggcctgccacttcagtcaactccactgaggagactgccactaca

gttgttgctgctgcctcctccaccagcatccctagctcaga

The obesity risk diagnosis kit of the present invention may appropriately include known elements for genetic diagnosis in addition to the above probe.

A second aspect of the present invention relates to a method for analyzing the risk of obesity onset. This method includes the steps of measuring the expression level of a *SYNOVIOLIN* gene in a sample collected from an organism as a subject and determining the risk of obesity onset using the expression level of the *SYNOVIOLIN* gene. The step of measuring the expression level of the *SYNOVIOLIN* gene is as described above. In order to determine the risk of obesity onset using the expression level of the *SYNOVIOLIN* gene, the above expression level may be compared to the expression level of the *SYNOVIOLIN* gene in a sample collected from a healthy individual. In addition, threshold values related to the expression levels of *SYNOVIOLIN* genes in samples of a plurality of obese individuals or non-obese individuals are determined, and the threshold values may be compared with each other.

The method for analyzing the risk of obesity onset may be automatically determined using a computer. The computer has an input/output unit, an operation unit, a control unit, and a storage unit, and these units are capable of exchanging information using a bus or the like. The storage unit stores a threshold value related to the expression level of the *SYNOVIOLIN* gene corresponding to a sample (e.g., blood, saliva, nail, hair). Information on the subject (ID etc.), information on the sample, and information on the expression level of the *SYNOVIOLIN* gene are input from the input/output unit to the computer. Then, the control unit reads out an operation program stored in the storage unit, reads out a threshold value corresponding to the sample from the storage unit, and allows the operation unit to compare the expression level of the synoviolin gene with the threshold value. Then, based on the comparison result, the operation unit obtains an analysis result of the risk of obesity onset. The control unit sends the obtained analysis result to the storage unit as appropriate, and the result is stored therein and is output from the input/output unit. In this way, the risk of obesity onset of the subject is determined.

### Examples

Hereinafter, the present invention will be specifically described with reference to examples. The present invention is not limited to the following examples.

### RNA Collection and Real-Time PCR

Blood was collected from 33 healthy individuals and lymphocytes were collected using the Ficoll separation method. Total RNA was generated by ISOGEN (Nippon Gene Co., Ltd.) using the collected lymphocytes. Table 1 shows information on age, gender, height, and BMI of healthy individuals (healthy donors) in the examples.

**[Table 1]**

| | All healthy individuals | Healthy individual (BMI < 25 kg/m²) (n=25) | Healthy individual (BMI ≧25 kg/m²) (n=8) | P |
|---|---|---|---|---|
| Age | 38.8 | 38.0 | 41.4 | 0.4917 |
| Number of women (%) | 22 (66.7) | 16 (64) | 6 (75) | 0.5716 |
| Weight(kg) | 58.6 | 54.1 | 72.7 | P < 0.00001 |
| Height(m) | 1.6 | 1.6 | 1.6 | 0.5013 |
| BMI | 22.9 | 20.9 | 28.8 | P < 0.0000001 |

1 µg of total RNA was subjected to reverse transcription with ReverTra (manufactured by Toyobo Co., Ltd.) using random primers. Then, the expression levels of the *SYNOVIOLIN* gene and trademark body stress-associated gene were verified by real-time PCR using Light Cycler 480 Probes Master (manufactured by Roche). Ribosomal protein large P0 (*RPLP0*) was used as an internal control.

The primers and probes used in the examples were as follows.

SYVN 1: (synoviolin gene)
Forward primer: 5'-ccagtacctcaccgtgctg-3 (SEQ ID NO: 2)
Reverse primer: 5'-tctgagctagggatgctggt-3' (SEQ ID NO: 3)
The probe No. 16 of the Universal ProbeLibrary (Roche) was used.

### ATF6:

Forward primer: 5'-gcagaaggggagacacattt-3' (SEQ ID NO: 4)
Reverse primer: 5'-tgtggtcttgttatgggtggt-3' (SEQ ID NO: 5)

The probe No. 62 of the Universal Probe Library (Roche) was used.

### XBP1:

Forward primer: 5'-ggagttaagacagcgcttgg-3' (SEQ ID NO: 6)
Reverse primer: 5'-cactggcctcacttcattcc-3' (SEQ ID NO: 7)

The probe No. 37 of the Universal ProbeLibrary (Roche) was used.

### IRE1:

Forward primer: 5'-gaagcatgtgctcaaacacc-3' (SEQ ID NO: 8)
Reverse primer: 5'-tctgtcgctcacgtcctg-3' (SEQ ID NO: 9)

The probe No. 50 of the Universal ProbeLibrary (Roche) was used.

### eIF2a:

Forward primer: 5'-gaagctaagaaagctgcaaagc-3' (SEQ ID NO: 10)
Reverse primer: 5'-cagtgtttcgtggtgtgctc-3' (SEQ ID NO: 11)

The probe No. 43 of the Universal ProbeLibrary (Roche) was used.

### GRP78:

Forward primer: 5'-catcaagttcttgccgttca-3' (SEQ ID NO: 12)
Reverse primer: 5'-ttcaggagcaaatgtctttgttt-3' (SEQ ID NO: 13)

The probe No. 10 of the Universal ProbeLibrary (Roche) was used.

### RPLPO:

Forward primer: 5'-gcagaaggggagacacattt-3' (SEQ ID NO: 14)
Reverse primer: 5'-tgtggtcttgttatgggtggt-3' (SEQ ID NO: 15)

The probe No. 62 of the Universal ProbeLibrary (Roche) was used.

A highly efficient reverse transcriptase (ReverTra Ace (registered trademark), manufactured by Toyobo Co., Ltd.) was used for RT-PCR.

The conditions of RT-PCR were as follows. In RT-PCR, the conditions such as temperature setting were determined based on the instruction manual of the used kit.

1 µg of total RNA and RNase-free water were combined to obtain a total of 10 µL, and the mixture was incubated at 65°C for 5 minutes, and then cooled on ice for 5 minutes.

Thereafter, the following raw materials were added:

| | |
|---|---|
| 5 × buffer | 4 µL |
| 10 mM dNTPs mixture | 2 µL |
| ReverTra Ace (R) | 1 µL |
| RNase inhibitor | 0.5 µL |
| Random primer (25 pmol/µL) | 1 µL |
| RNase-free water | 1.5 µL |
| RNA solution | 10 µL |
| Total | 20 µL |

The mixture was incubated at 30°C for 10 minutes, incubated at 42°C for 30 minutes, and incubated at 99°C for 5 minutes.

Statistical analysis was performed on the obtained expression levels using the Excel Statistical software 2012 (manufactured by SSRI, Japan). The RNA expression levels of genes were divided into subjects with a body mass index (BMI) of 25 kg/m² or more and a BMI of 25 kg/m² or less. Then, a statistically significant difference between the expression levels was examined by the unpaired Student's t-test. At that time, when the P value was 0.05 or less, it was judged that there was the statistically significant difference.

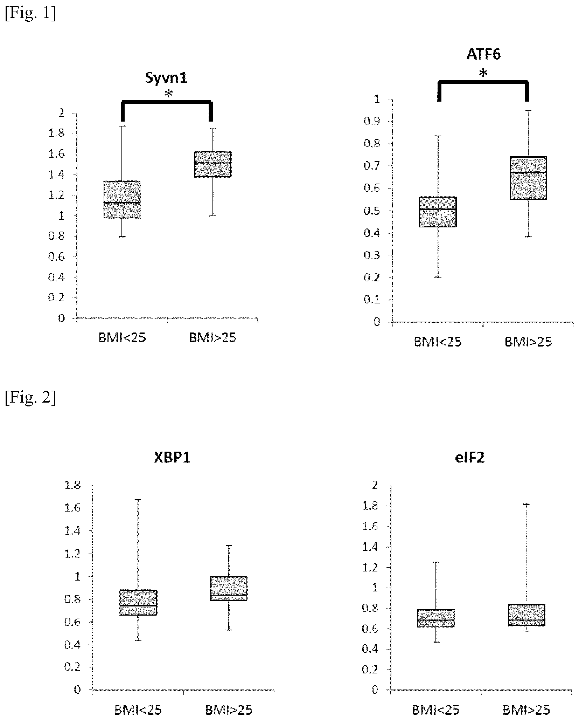

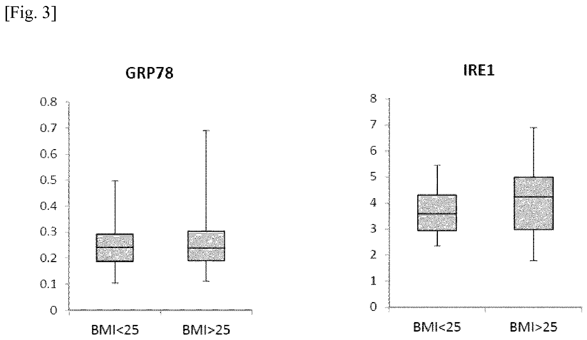

Figs. 1 to 3 are box plots showing the expression levels of RNAs. Fig. 1 is a box plot showing the expression levels of the *SYNOVIOLIN* gene and the *ATF 6* gene. Fig. 2 is a box plot showing the expression levels of the *XBP1* gene and the *elF2* gene. Fig. 3 is a box plot showing the expression levels of the *GRP78* gene and the *IREI1* gene.

As shown in Fig. 1, between the subject with a body mass index (BMI) of 25 kg/m² or more and the subject with a BMI of 25 kg/m² or less, statistically significant differences were observed in the expression levels of the *SYNOVIOLIN* gene and the *ATF6* gene. Hence, it is found that the expression level of the *SYNOVIOLIN* gene in the subject with a BMI of 25 kg/m² or more is higher than that in the subject with BMI of 25 kg/m² or less, with a statistically significant difference. Hence, it is shown that the risk of becoming genetically obese can be evaluated by determining the expression level of the *SYNOVIOLIN* gene.

### Industrial Applicability

The present invention can be used in the genetic diagnostic equipment field and the diagnostic industry.

### [Sequence List Free Text]

SEQ ID NO: 2: primer
SEQ ID NO: 3: primer
SEQ ID NO: 4: primer
SEQ ID NO: 5: primer
SEQ ID NO: 6: primer
SEQ ID NO: 7: primer
SEQ ID NO: 8: primer
SEQ ID NO: 9: primer
SEQ ID NO: 10: primer
SEQ ID NO: 11: primer
SEQ ID NO: 12: primer
SEQ ID NO: 13: primer
SEQ ID NO: 14: primer
SEQ ID NO: 15: primer

## Claims

1. An obesity risk diagnosis kit comprising a probe for identifying a *SYNOVIOLIN* gene as an obesity marker.

2. A method for analyzing a risk of obesity onset, comprising the steps of:
measuring an expression level of a *SYNOVIOLIN* gene in a sample collected from an organism as a subject; and
determining the risk of obesity onset using the expression level of the *SYNOVIOLIN* gene.
